# EUROPEAN PATENT APPLICATION

(11) **EP 4 123 020 A1**
(43) Date of publication of application: **25.01.2023**
(21) Application number: 20926074.4
(22) Date of filing: 19.03.2020
(51) Int. Cl.: C12N 7/00

(54) **NEW BIOLOGICAL NITRIFICATION-INHIBITING BACTERIOPHAGES AND USE OF SAME**

(71) Applicant: Fertinagro Biotech, S.L., 44195 Teruel (ES)
(72) Inventor: ATARES REAL, Sergio, 44195 Teruel (ES); ROMERO LOPEZ, Joaquin, 44195 Teruel (ES); SALAET MADORRAN, Ignasi, 44195 Teruel (ES); FERRER GINES, Maria, 44195 Teruel (ES); YANCE CHAVEZ, Tula del Carmen, 44195 Teruel (ES); FUERTES DOÑATE, Carlos, 44195 Teruel (ES); CABALLERO MOLADA, Marcos, 44195 Teruel (ES); QUIRÓS FERNÁNDEZ, Pablo, 44195 Teruel (ES); MUNISE PÉREZ, María Teresa, 44195 Teruel (ES)
(74) Representative: González López-Menchero, Álvaro Luis
(86) International application number: PCT/ES2020/070192
(87) International publication number: WO 2021/186088

(57) **Abstract**

The invention provides new bacteriophages and variants thereof capable of inhibiting the proliferation and/or growth of nitrifying microorganisms present in the soil, responsible for the nitrification of agricultural soils, in particular capable of inhibiting the proliferation and/or growth of ammonia-oxidising bacteria, such as *Nitrosomonas sp, Nitrosomonas aestuarii, Nitrosomonas communis, Nitrosomonas europaea, Nitrosomonas eutropha, Nitrosomonas halophila, Nitrosomonas marina, Nitrosomonas nitrosa, Nitrosomonas oligotropha, Nitrosomonas ureae, Nitrosospira sp, Nitrosospira multiformis, Nitrosospira tenuis.*

## Description

The present invention concerns new nitrification-inhibiting bacteriophages and their use.

More specifically, the invention provides new nitrification-inhibiting bacteriophages, or variants thereof, capable of inhibiting the proliferation and/or growth of nitrifying microorganisms present in the soil, responsible for the nitrification of agricultural soils, in particular capable of inhibiting the proliferation and/or growth of ammonia-oxidising bacteria (AOB, such as *Nitrosomonas sp, Nitrosomonas aestuarii, Nitrosomonas communis, Nitrosomonas europaea, Nitrosomonas eutropha, Nitrosomonas halophila, Nitrosomonas marina, Nitrosomonas nitrosa, Nitrosomonas oligotropha, Nitrosomonas ureae, Nitrosospira sp, Nitrosospira multiformis, Nitrosospira tenuis.*

Nitrogen (N) provided through mineral and organic fertilisers has had a very beneficial impact on society, allowing for a major increase in food production (Erisman et al., (2008) "How a century of ammonia synthesis changed the world", Nature Geoscience1:636-639). The synthesis of ammonia from nitrogen in the air and its subsequent transformation into nitrogen fertilisers, together with the use of other means of production, has boosted the yield of most crops to today's levels.

However, in recent years, advances in the understanding of the constituent processes of the nitrogen cycle have also raised concerns about the difficulty of increasing food production without generating significant losses of reactive nitrogen (nitrate, nitrogen oxides, ammonia) in intensive systems, both to water bodies and to the atmosphere (Galloway et al., "Toxicity of Nitrification Inhibitors on Dehydrogenase Activity in Soils", International Journal on Advanced Science, Engineering and Information Technology, vol. 1, no. 1, pp. 98-103, 2011).

It is estimated that agriculture generates 56-70% of the nitrous oxide (N₂O) emitted into the atmosphere, and the contribution of this greenhouse gas (GHG) to the carbon footprint of agricultural food crops is also very high (Smith et al., 2007, "Greenhouse gas mitigation in agriculture", Cambridge University Press, Cambridge, United Kingdom and New York).

Most modern agricultural systems have become high nitrification environments where nitrification is so rapid that most of the NH₄⁺ from external inputs (mineral fertilisers) is nitrified within a few weeks (often 2 weeks) (Subbarao et al. 2006, "Scope and strategies for regulation of nitrification in agricultural systems - challenges and opportunities", Critical Reviews in Plant Science 25:303-335). Nitrate is vulnerable to losses either through leaching or denitrification (Sahrawat, 2008, "Factors affecting nitrification in soils", Communications in Soil Science and Plant Analysis. 39: 1436-1446).

Due to its high solubility and its negative ionic nature, nitrate nitrogen has a very low retention coefficient in the soil. The organic matrix of the soil, which is also negatively charged, has no capacity to retain this compound. Thus, it travels through the soil at a high speed, leaving little time to be absorbed by plants or fixed by soil micro-organisms. Within a short time, it moves to deep areas, where it is no longer accessible to root uptake and is eventually lost to groundwater bodies. Because of this characteristic, its use is beginning to be restricted or even banned in areas considered vulnerable.

In contrast, ammoniacal nitrogen has a positive charge and is stable in the soil complex. It is retained with very little loss through leaching. This greatly increases its residence time and, consequently, the time during which it is available to plants. Moreover, contamination of groundwater bodies is reduced due to the low leaching loss. The main point to be taken into account in the use of ammonium is that it is gradually converted to nitrate nitrogen in the soil by micro-organisms with nitrifying capacity.

In this respect, the nitrification process occurs in two steps, with the first step oxidising ammonia to nitrite and the second step oxidising nitrite to nitrate. With some exceptions, these two steps are carried out by different micro-organisms. The vast majority of bacteria involved in the two steps are autotrophic, being able to manufacture organic molecules using energy obtained from inorganic sources, in this case ammonia or nitrite.

In the first step of nitrification, the ammonia oxidation step, two very different groups of micro-organisms can be distinguished.

The first group is known as ammonia-oxidising bacteria (AOB) and includes several types of bacteria that obtain their energy by generating a reducing potential from the oxidation of ammonia, using this energy to fix carbon dioxide (Bock and Wagner, 2006, "Oxidation of inorganic nitrogen compounds as an energy source", Dworkin M, Falkow S (eds). The Prokaryotes Springer Verlag: New York; 457-495).

In general, these AOB are considered obligate autotrophic organisms, i.e. they can only grow by fixing their own CO₂ from the Calvin cycle. Ammonia is their only source of energy, their main metabolic product is nitrite and the reaction they carry out is shown in equation (1).

NH₃ + O₂ → NO₂⁻ + 3H⁺ + 2e- (1)

Nitrosomonas is the most frequently identified genus associated with this step, but other genera such as Nitrosococcus and Nitrosospira are capable of this step. There are also subgenera such as Nitrosolobus and Nitrosovibrio that can autotrophically oxidise ammonia (Watson et al., 1981, "The family nitrobacteraceae, in The Prokaryotes: A Handbook on Habitats, Isolation and Identification of Bacteria", ed. M. P. Starr et al., pp. 1005-1022, Springer, New York).

The second group of ammonia-oxidising microorganisms was isolated in 2005 (Könneke et al., 2005, "Isolation of an autotrophic ammonia-oxidizing marine archaeon", Nature; 437:543-546). These microorganisms are not bacteria, but archaea, specifically Ammonia-Oxidising Archaea (AOA). Like AOBs, AOAs oxidise ammonia to nitrite, but the enzymatic pathways are quite different and so is the overall reaction, as shown in equation (2).

NH₃ + 1.5 O₂ → NO₂⁻ + H₂O + H+ (2)

AOAs are also thought to be predominantly autotrophic, but fix CO₂ using the 3-hydroxypropionate/4-hydroxybutyrate pathway (Walker et al., 2010, "Nitrosopumilus maritimus genome reveals unique mechanisms for nitrification and autotrophy in globally distributed marine crenarchaea", Proc Natl Acad Sci USA; 107:8818-8823). The biodiversity of AOAs with the capacity to perform this function is much broader than in the case of AOBs. Thus, this ability has been identified in an entire phylum of Archaea, the Thaumarchaeota (Treusch et al., 2005, "Novel genes for nitrite reductase and Amo-related proteins indicate a role of uncultivated mesophilic crenarchaeota in nitrogen cycling", Environ Microbiol 7: 1985-1995).

In view of the variety of microorganisms capable of carrying out this first step of nitrification, many studies have focused on their distribution in multiple habitats. It has been shown that the abundance and diversity of AOA and AOB is clearly affected by environmental factors such as pH (Li et al., 2015, "pH regulates ammonia-oxidizing bacteria and archaea in paddy soils in Southern China", Appl Microbiol Biot, 1-11 (2015); Zhang et al, 2012, "Ammonia-oxidizing archaea have more important role than ammonia-oxidizing bacteria in ammonia oxidation of strongly acidic soils", ISME J 6, 1032-1045), salinity (Bernhard et al., 2010, "Abundance of ammonia-oxidizing archaea and bacteria along an estuarine salinity gradient in relation to potential nitrification rates", Appl Environ Microb 76, 1285-1289; Mosier & Francis, 2008, "Relative abundance and diversity of ammonia-oxidizing archaea and bacteria in the San Francisco Bay estuary", Environ Microbiol 10, 3002-3016), the amount of ammonia nitrogen and organic matter (Liu et al., 2013, "Spatial distribution and factors shaping the niche segregation of ammonia-oxidizing microorganisms in the Qiantang River, China", Appl Environ Microb 79, 4065-4071; Verhamme et al., 2011, "Ammonia concentration determines differential growth of ammonia-oxidising archaea and bacteria in soil microcosms", ISME J 5, 1067-1071; Sun et al., "Distribution and abundance of archaeal and bacterial ammonia oxidizers in the sediments of the Dongjiang River, a drinking water supply for Hong Kong", Microbes Environ 28, 457 (2013)).

Since ammonia is the substrate for oxidation, the amount of ammonium is considered to be a major factor affecting the distribution of this type of micro-organisms. Thus, the studies of Verhamme (*above*) and many subsequent studies have shown that, in (non-acidic) agricultural soils with relevant amounts of ammonium, AOB are the dominant micro-organisms in terms of nitrification.

This first step of the nitrification process (oxidation of ammonia to nitrite) is the limiting step, and therefore, the one that will mark the rate at which ammonium is transformed into nitrate, with all the problems that this entails in an agricultural environment. However, this is not the only associated negative effect, since, in addition to the net production of nitrite, AOBs are also capable of producing nitrous oxide (N₂O), a gas with 298 times more greenhouse capacity than CO₂.

Fertiliser technology has tried to slow down the rate of nitrification by developing fertilisers that include chemical compounds capable of inhibiting nitrification in socalled stabilised fertilisers. Such compounds deactivate the enzyme responsible for the first stage of nitrification (ammonium monooxygenase, AMO), keeping NH₄⁺ in the soil for longer (Ruser and Schulz, 2015, "The effect of nitrification inhibitors on the nitrous oxide (N2 O) release from agricultural soils - a review", J. Plant Nutr. Soil Sci. 178, 171-188; Gilsanz et al., 2016, "Development of emission factors and efficiency of two nitrification inhibitors, DCD and DMPP", Agriculture, Ecosystems and Environment 216, 1-8.). Dicyandiamide (DCD) and 3,4-dimethylpyrazole phosphate (DMPP), which have been used in recent years, have proven to be efficient in reducing nitrogen oxides emissions under different climatic conditions. This effect on nitrification produced by the two inhibitors is correlated with a reduction in the leaching of dissolved nitrogen when either of them is used, as well as a reduction in the emission of pollutant gases.

In this respect, see for example EP1378499B1, describing the use of the nitrification inhibitor 3,4-dimethylpyrazole phosphate in combination with nitrogen fertilisers or EP1546285A1, describing the use of dicyandiamide as a nitrification inhibitor.

However, the use of chemical inhibitors can also present problems, due to the possibility of their uptake by plants while they remain in the soil, and it is currently a major challenge to understand how these inhibitors degrade in the soil and whether such products are passed on to the plant. Marsden et al. ("Plant acquisition and metabolism of the synthetic nitrification inhibitor dicyandiamide and naturally-occurring guanidine from agricultural soils", Plant and Soil, 395 (1-2), 201-214, 2015) demonstrated that DCD can pass to and be partially metabolised in grass plants, being a possible vehicle for its transmission to the food chain. In the case of DMPP, it has also been shown that it can remain in the soil for longer than its inhibitory effect (Guardia et al., "Fate of 15N-labelled ammonium nitrate with or without the new nitrification inhibitor DMPSA in an irrigated maize crop", Soil Biology & Biochemistry. 116: 193-202, 2018) which can be negative if taken up by the plant.

The effect of different inhibitors on the soil microbial assemblage could also be demonstrated by analysis of enzyme activities such as dehydrogenase activity or dimethylsulfoxide reductase activity (Tindaon et al., 2010; Tindaon et al., 2011, "Side Effects of Nitrification Inhibitors on Non Target Microbial Processes in Soils",. Jurnal TANAH TROPIKA (Journal of Tropical Soils). 16. 7-16. 10.5400/jts.2011.16.1.7.)

Bacteriophages are considered non-living biological entities consisting of a nucleic acid enveloped by a protein capsid. As obligate parasites of bacteria, they are unable to reproduce without their host. The first phage therapy proposed to treat infections was as early as 1919, almost a decade before the discovery of penicillin (Chanishvili N., "Phage therapy--history from Twort and d'Herelle through Soviet experience to current approaches", Adv Virus Res 2012; 83: 3-40). Later, with the development of antibiotics, such strategies were abandoned due to the enormous technical difficulties at the time. Today, with technological developments and the problems associated with antibiotics, it is re-emerging as a tool with great potential.

Bacteriophages are simple and incredibly diverse. They are widely distributed in all ecosystems, in particular it is estimated that on average there are 1.5×10⁸ phages/g soil (Kevin E. et al; 2003, "Elevated Abundance of Bacteriophage Infecting Bacteria in Soil", Applied and Environmental Microbiology, p. 285-289 Vol. 69) and up to 10¹⁰ phages/g can be counted in certain soils.

The advantages of phage therapy over antibiotics in medicine can be extrapolated to the use of this type of technology versus inhibitory molecules applied in other fields, such as nitrification inhibitors in agriculture.

Thus, bacteriophages are highly specific, i.e. each virus has limited infection capacity from a few species to only a few strains of the same species. This feature is very important, as it greatly reduces possible side effects by not greatly affecting the non-targeted microbiological flora.

Phage therapy is also self-dosed. The application of chemical inhibitors requires minimum concentrations of inhibitory substances and stability of the inhibitory substances to continue to perform their function. Phages are self-replicating entities in their target bacteria. The presence of few bacteria will mean few infections and limited generation of new phages. The more bacteria there are and the more they are multiplying, the more pronounced the parasitisation by phages will be and the more the number of viruses will grow exponentially, multiplying the desired effect.

Moreover, the use of bacteriophages does not leave polluting chemical residues, as they are still organic matter. These entities have a limited half-life in the soil in the absence of their host. This half-life is subject to different variables such as temperature, humidity, soil composition, etc.

There is abundant documentation on the use of this type of technology especially in the field of pest control, where it has been most developed. There is a well-recognised need to develop new environmentally friendly control strategies to combat bacterial crop diseases. Current control measures involving the use of traditional chemicals or antibiotics are losing their effectiveness due to the natural development of bacterial resistance to these agents. In addition, there is a growing awareness that their use is not environmentally friendly.

Bacteriophages have received increasing research interest in recent years as a realistic and environmentally friendly means of controlling bacterial diseases in crops, with some bacteriophage-based products already available on the market. This biological control with bacteriophages has advantages over chemical controls, as tailor-made bacteriophage cocktails can be adapted to target specific disease-causing bacteria, easily accommodating bacterial resistance that may develop over time.

The use of phages as a tool can initially be presented as a forced situation promoted in the soil environment. The study of interactions between viruses and their target bacteria in different habitats reveals that such ecological interactions are naturally abundant in different ecosystems. Phages are considered to be the most abundant biological entity on Earth and play a key role in the regulation of bacterial populations. For example, phages are responsible for killing approximately 20-40% of all bacteria on the sea surface every 24 hours (Wittebole X. et al; 2014, "A historical overview of bacteriophage therapy as an alternative to antibiotics for the treatment of bacterial pathogens", Virulence; 5: 226-235). The effect of these bacteriophage-bacteria interactions at the soil level is also widely described. Soil provides an incredible range of niches that are occupied by many species of micro-organisms, leading to a similar diversity in the variety of viruses that parasitise these micro-organisms. In agricultural soils, viral abundance is significantly correlated with bacterial abundance (Williamson et al., 2007, "Incidence of lysogeny within temperate and extreme soil environments", Environ Microbiol 9:2563-2574). This indicates that the presence and abundance of susceptible hosts is a key factor controlling viral abundance. When host organisms are present, particularly in large numbers, new viruses can be continuously produced and released into the soil matrix through lytic infections. In the absence of susceptible hosts, the abundance of extracellular viruses is controlled by the physical and chemical properties of the soil environment. The effect of this virus-bacteria balance has been demonstrated, for example, with phages parasitising bacteria of the genus *Rhizobium* (Kleczkowska J; 1971, "Genetic changes in rhizobium bacteria and in their bacteriophages during coexistence", Plant Soil 35(1):47-56). Another demonstration of the high frequency of such soil infections and their ecological importance was shown in experiments carried out by Allen et al. in 2010. In habitats with high amounts of soluble organic carbon, the microbiological mass does not grow even if more consumable substrates are incorporated; in contrast, the application of anti-phage substances generates a substantial increase in the number of micro-organisms. This shows that these populations in the soil were in a controlled equilibrium due to the permanent presence of the phages.

Thus, the invention aims to avoid the use of chemical compounds and to eliminate the disadvantages of known prior art products, which may entail adverse effects in preventing nitrification of agricultural soils, by providing new nitrification inhibiting bacteriophages according to SEQ ID NO: 1, herein referred to as Phi-NF1 bacteriophages (deposited in the German DSZ collection under deposit number DSM 33308), capable of inhibiting the proliferation and/or growth of specific nitrifying bacteria present in soil, in particular capable of inhibiting the proliferation and/or growth of ammonia-oxidising bacteria (AOB), in particular and preferably *Nitrosomonas sp, Nitrosomonas aestuarii, Nitrosomonas communis, Nitrosomonas europaea, Nitrosomonas eutropha, Nitrosomonas halophila, Nitrosomonas marina, Nitrosomonas nitrosa, Nitrosomonas oligotropha, Nitrosomonas ureae, Nitrosospira sp, Nitrosospira multiformis, Nitrosospira tenuis.*

The invention also covers variants of said bacteriophages, provided that said variants retain the phenotypic characteristics of the original bacteriophage. In this context, a variant of the bacteriophage of SEQ ID NO: 1 means a variant having a sequence identity, as compared to SEQ ID NO: 1, of at least 70%, where the variant retains the phenotypic characteristics of the bacteriophage of SEQ ID NO: 1 from which it is derived.

It is likewise an object of the invention to use the bacteriophage according to SEQ ID NO: 1 , or a variant thereof as defined herein, herein referred to as bacteriophage Phi-NF1 (deposited in the German DSZ collection under deposit number DSM 33308), to inhibit the proliferation and/or growth of specific nitrifying bacteria present in soil, in particular to inhibit the proliferation and/or growth of ammonia-oxidising bacteria (AOB), in particular and preferably *Nitrosomonas sp, Nitrosomonas aestuarii, Nitrosomonas communis, Nitrosomonas europaea, Nitrosomonas eutropha, Nitrosomonas halophila, Nitrosomonas marina, Nitrosomonas nitrosa, Nitrosomonas oligotropha, Nitrosomonas ureae, Nitrosospira sp, Nitrosospira multiformis, Nitrosospira tenuis.*

Thus, in a preferred embodiment of the use of the bacteriophage Phi-NF1 according to SEQ ID NO: 1, or a variant thereof as defined herein, it is applied by fertigation at a dose of between 10⁸ and 10¹³ Phi-NF1 units/litre of irrigation water.

Similarly, Phi-NF1 bacteriophage according to SEQ ID NO: 1, or a variant thereof as defined herein, can be formulated in conjunction with liquid or solid nitrogen fertilisers at a dose of between 10¹¹ and 10¹⁶ Phi-NF1 units/kg nitrogen.

The invention is described below on the basis of the following tests and examples of embodiments thereof and with reference to the following figures, wherein:
- Fig. 1:: Graph showing the variation of pH in an in vitro culture, infective capacity of phage 1 N.e;
- Fig. 2:: Graph showing the variation of pH in an in vitro culture, infective capacity of phage 2 N.e;
- Fig. 3:: Graph showing the pH variation in an in vitro culture, infective capacity of phage 3 N.m;
- Fig. 4:: Graph showing pH variation in an in vitro culture, infective capacity of phage 4 N.m;
- Fig. 5:: Transmission electron microscopy (TEM) image showing phage morphology on *Nitrosomonas europaea* (a) and *Nitrosospira multiformis* (b);
- Fig. 6A:: Reduction of the amount of ammonium in a culture of *Nitrosomonas europaea* infected or not with Phi-NF1;
- Fig. 6B:: Reduction of the amount of ammonium in a culture of *Nitrosospira multiformis* infected or not with Phi-NF1;
- Fig. 6C:: Reduction of the amount of ammonium in a *Nitrosomonas nitrosa* culture infected or not with Phi-NF1;
- Fig. 6D:: Reduction of the amount of ammonium in a culture of *Nitrosomonas communis* infected or not with Phi-NF1;
- Fig. 7:: Result of the *in vivo* assay of the activity of the phage Phi-NF1 as a biological nitrification inhibitor: A) Control soil treated with ammonium sulphate alone; B) Soil treated with ammonium sulphate + Phi-NF1.

### Bacteria and culture media and obtaining virus concentrates from natural samples

### a. Bacteria:

The bacterial species *Nitrosomonas europaea* (ATCC 25978) and *Nitrosospira multiformis* (ATCC 25196) were used as hosts for lytic virus isolation. Subsequently, after virus isolation, other AOB, e.g. *Nitrosomonas nitrosa* (DSM 28438) and *Nitrosomonas communis* (DSM 28436), were also used to test the phage infection spectrum.

### b. Culture medium (recommended by the German Collection of Cultures DSMZ):

Culture medium, OAB: 535 mg NH₄Cl, 54 mg KH₂PO₄, 74 mg KCl, 49 mg MgSO₄ × 7 H₂O, 147 mg CaCl₂ × 2 H₂O, 584 mg NaCl, 1 ml trace element solution, 1,000 ml distilled water.

The culture medium was sterilised in an autoclave and the pH was adjusted to 7.8 - 8.2 with sterile 10% KHCO₃ solution.

Culture conditions: The culture is carried out in the dark at a temperature of 30°C and a constant agitation of 60 rpm.

### c. Obtaining mixtures of virus concentrates from natural samples:

The natural samples for obtaining viral concentrates were urban wastewater samples from municipal wastewater treatment plants (an environment where a high presence of nitrifying bacteria has been described), both incoming wastewater (ARE) (untreated) and outgoing wastewater (ARS) (after secondary treatment in a treatment plant).

In order to obtain a viral concentrate for subsequent testing against nitrifying bacteria, inlet wastewater samples or ARE and outlet wastewater samples or ARS were pooled. To obtain the viral fraction from these samples and to concentrate the phage particles, the following steps were followed:
- Centrifugation at 200×g for 10 minutes and recovery of the supernatant to remove organic matter and bacterial growth;
- Filtering of the supernatant through PES filters of 0.22 µm pore size and low protein absorption;
- Concentration of the viral fraction of wastewater using a polyethylene glycol viral particle concentration protocol.

After this process, two samples of virus concentrates were obtained, one from the wastewater entering the treatment plant (cvARE) and one from the wastewater leaving the treatment plant (cvARS).

### Isolation of virulent bacteriophages against nitrifying bacteria

Nitrifying bacteria are slow-growing micro-organisms (22-28 days) and never reach sufficient growth to cause quantifiable turbidity in the liquid culture medium.

Therefore, the bacterial growth was monitored by measuring the pH. As mentioned, the culture medium is at a pH between 7.8 and 8.2, which is ideal for the development of these microorganisms.

As the bacterial population increases in a closed system, and due to ammonium nitrification, the pH of the medium becomes more acidic, until it reaches a point incompatible with the life of the micro-organisms themselves. Thus, when the pH begins to fall below 7, it is brought back to pH 7.8-8.2 by adding a sterile 10% KHCO₃ solution of.

To carry out the phage isolation process, the following four scenarios are considered:
1- Infection of *Nitrosomonas europaea* with cvARE
2- *Infection of Nitrosomonas europaea* with cvARS
3- Infection of *Nitrosospira multiformis* with cvARE
4- Infection of *Nitrosospira multiformis* with cvARS

To an active growth of 25 ml bacteria (*N*. *europaea.* or *N. multiformis.* ), 200 µl of a virus concentrate (cvARE or cvARS) was added, each combination being assessed in triplicate (t1, t2, t3). In the case of a positive infection, the bacteriophages will lyse the bacteria, causing a complete halt in the pH decrease (or even a slight increase in pH due to the rupture of the bacterial walls). Said halt in pH decrease contrasts with actively growing controls, the pH of which continues to decrease normally. Viral concentrates that do not contain lytic bacteriophages for these micro-organisms will have no effect on the bacterial population and therefore the pH will continue to decrease as in the control situation.

The following tables 1 and 2 show the evolution of the experiments to find infective bacteriophages for *Nitrosomonas europaea* and *Nitrosospira multiformis.* Both tables show the pH over 7 days in various actively growing cultures of the species *Nitrosomonas europaea* (N.e) and *Nitrosospira multiformis* (N.m). At time 0, concentrated virus samples (cvARE or cvARS) are added to the cultures (except in control situations) in order to achieve infection in one of the cultures and thus obtain one or more bacteriophages virulent to these ammonium-oxidising species. At time 4, the pH of all cultures is readjusted back to pH 8. In the case of phage infection, the bacteria will be dead, so the pH will either remain stable or rise slightly due to bacterial lysis.

**Table 1 Evolution of Nitrosomonas Europaea and Nitrosospira multiformis cultures treated with concentrated samples of natural phages from treatment plant inlet wastewater.**

| | **N.e Control** | **N.e + [cvARE] t1** | **N.e + [cvARE] t2** | **N.e + [cvARE] t3** | **N.m Control** | **N.m + [cvARE] t1** | **N.m + [cvARE] t2** | **N.m + [cvARE] t3** |
|---|---|---|---|---|---|---|---|---|
| | **pH** | | | | | | | |
| **Day 0** | 8 | 8 | 8 | 8 | 8 | 8 | 8 | 8 |
| **Day 4** | 6.33→8 | 7.54→8 | 7.53→8 | 6.80→8 | 6.11→8 | 6.34→8 | 6.23→8 | 5.91→8 |
| **Day 7** | 6.65 | 8.12 | 8.11 | 6.60 | 6.32 | 7.10 | 6.44 | 6.71 |

**Table 2 Evolution of Nitrosomonas Europaea and Nitrosospira multiformis cultures treated with concentrated samples of natural phages from treatment plant outlet wastewater.**

| **pH** | **N.e Control** | **N.e + [cvARS] t1** | **N.e + [cvARS] t2** | **N.e + [cvARS] t3** | **N.m Control** | **N.m + [cvARS] t1** | **N.m + [cvARS] t2** | **N.m + [cvARS] t3** |
|---|---|---|---|---|---|---|---|---|
| **Day 0** | 8 | 8 | 8 | 8 | 8 | 8 | 8 | 8 |
| **Day 4** | 6.86→8 | 7.4→8 | 6.75→8 | 6.58→8 | 7.66→8 | 7.79→8 | 7.79→8 | 7.44→8 |
| **Day 7** | 6.32 | 6.87 | 6.85 | 6.97 | 6.87 | 7.21 | 7.23 | 6.8 |

As can be seen in table 1, two of the triplicates of *Nitrosomonas europaea* infection have stabilised pH and pH does not change over time. This means that one or more lytic phages for this bacterium could be present in these t1 and t2 replicates from cvARE.

As can be seen in table 1, one of the triplicates of *Nitrosospira multiformis* infection has stabilised the pH at 7.1 compared to the control, which remained at 6.32. This means that one or more lytic phages for this bacterium could be present in this replicate (t1) from cvARE.

As can be seen in table 2, all three triplicates of *Nitrosomonas europaea* infection have reduced the rate of pH decline. This means that one or more lytic phages for this bacterium might be present in these t1, t2 and t3 replicates from cvARS.

As can be seen in table 2, two of the triplicates of *Nitrosospira multiformis* infection have stabilised the pH at 7.2 compared to the control which remained at 6.87. This means that one or more lytic phages for this bacterium could be present in these replicates (t1 and t2) from cvARS.

Since, in principle, it is impossible to know whether, in tests where infection has occurred, it is due to the presence of one infective phage or several, the aim is to identify the possible phages present in the different samples.

The ideal situation to isolate the possible viruses in each positive sample would be to use the technique of obtaining lysis plaques in solid medium. Once the possible viruses in each sample have been identified, the most virulent virus can be selected. This situation is not very feasible considering the growth rate of nitrifying bacteria in solid medium. Therefore, the individualisation of the most virulent virus from each sample in liquid medium by selection by repeated infection is proposed. In this method, viruses from one infection are used to re-infect new cultures. This process is repeated during different cycles, so that the most efficient viruses infecting the target bacterium will be more and more predominant in each new cycle. After a relevant number of cycles, only the most active virus will be in the sample.

To carry out this process, positive replicates of each of the combinations are mixed and filtered through a 0.22 µm filter to obtain pure phage suspensions. This results in 4 phage mixtures:
- Phage 1 N.e (from infection of *Nitrosomonas europaea with cvARE*)
- Phage 2 N.e (from infection of *Nitrosomonas europaea with cvARS*)
- Phage 3 N.m (from infection of *Nitrosomonas multiformis with cvARE*)
- Phage 4 N.m (from infection of *Nitrosomonas multiformis with cvARS*)

The samples Phage 1N.e and Phage 2N.e are used to make successive infections (5) of *Nitrosomonas europaea.* The samples Phage 3N.e and Phage 4 N.m are used to make successive infections (5) of *Nitrosospira multiformis.*

Once the most virulent bacteriophage was obtained from each sample, its infective capacity was demonstrated. Two cultures of *Nitrosomonas europaea* (N.e) and two cultures of *Nitrosospira multiformis* (N.m) of 50 ml each, both actively growing, were used.

These 4 cultures were separated into 2 cultures of 25 ml each, leaving one of them as a control and the other to be infected with the corresponding bacteriophage. The pH was monitored for 5 days.

A schematic diagram of the experiment is shown below:

Control 1 N.e
N.e. culture 1 (50 ml)
N.e + Phage 1N.e
Control 2 N.e
N.e. culture 2 (50 ml)
N.e + Phage 2N.e
pH monitoring for 5 days
Control 1 N.m
N.m. culture 1 (50 ml)
N.m + Phage 3N.m
Control 2 N.m
N.m. culture 2 (50
N.m + Phage 4N.m

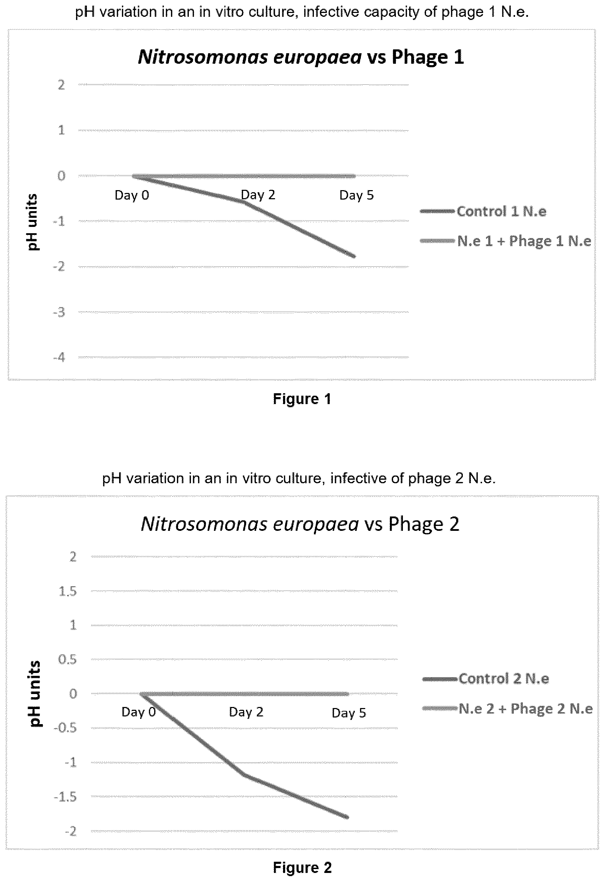

The results of this experiment are shown in figures 1 to 4.

As can be seen in Fig. 1, and in reference to the control, the bacteria begin to multiply, acidifying the medium, and causing a decrease of 1.78 pH points after 5 days. On the other hand, Phage 1 N.e. is able to infect the bacteria from day 0, causing bacterial death, and therefore, the non-acidification of the medium.

As can be seen in Fig. 2, and in reference to the control, the bacteria begin to multiply, acidifying the medium, and causing a decrease of 2.98 pH points after 5 days. On the other hand, Phage 2 N.e is able to infect the bacteria from day 0, causing bacterial death, and therefore, the non-acidification of the medium.

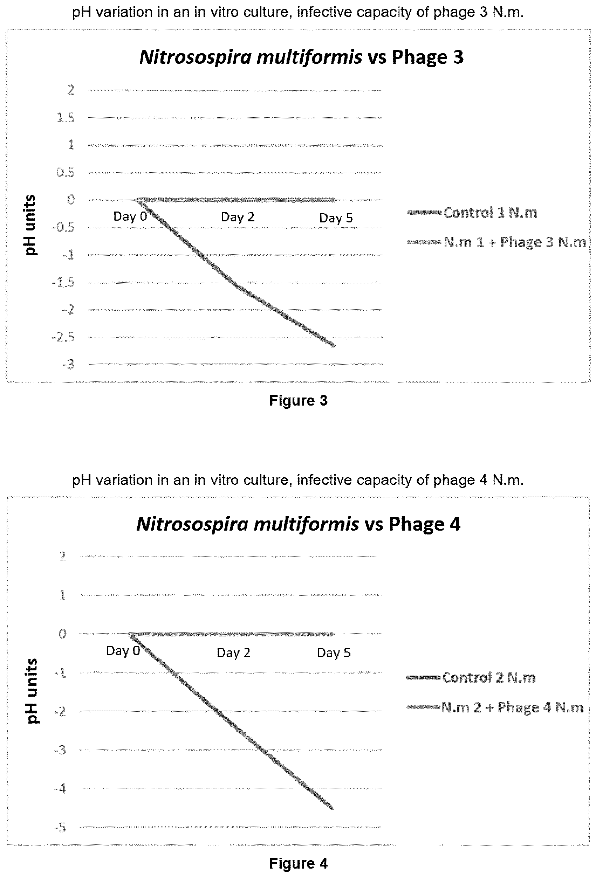

As can be seen in Fig. 3, and in reference to the control, the bacteria begin to multiply, acidifying the medium, and causing a decrease of 2.66 pH points after 5 days. On the other hand, Phage 3 N.m is able to infect the bacteria from day 0, causing bacterial death, and therefore, the non-acidification of the medium.

As can be seen in Fig. 4, and in reference to the control, the bacteria start to multiply, acidifying the medium, and causing a decrease of 4.5 pH points after 5 days. On the other hand, Phage 4 N.m is able to infect the bacteria from day 0, causing bacterial death, and therefore, the non-acidification of the medium.

### Electron microscopy

Once the virulence effect of the phages on *Nitrosomonas europaea* and *Nitrosospira multiformis* was observed, their morphology was checked under a transmission electron microscope (TEM).

To obtain a sufficient number of phages to be visible on TEM, 100 ml of each phage was concentrated by protein concentrators to 1 ml.

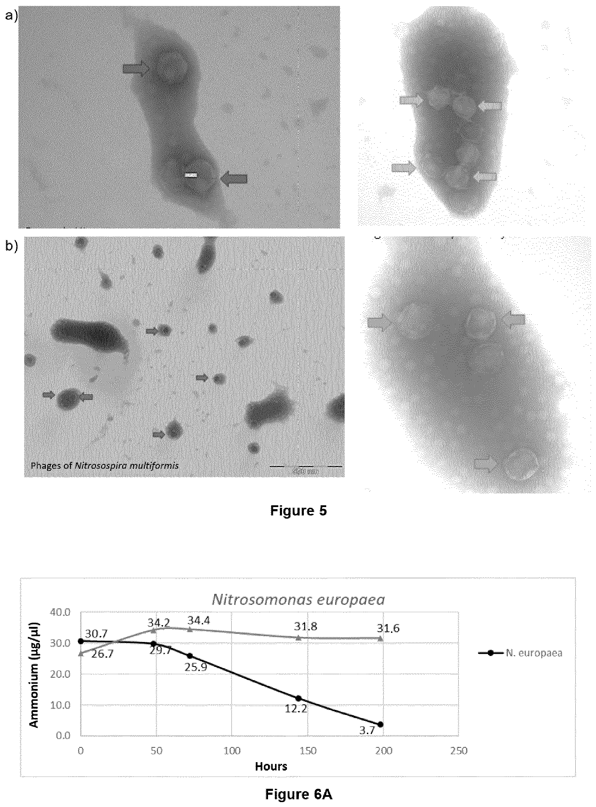

The images obtained in the TEM were those of bacteriophages of the family Podoviridae, with an icosahedral capsid of about 50 nm in diameter and a short tail. All 4 bacteriophages had the same morphology (Fig. 5).

### Sequencing

Phage DNA extraction was performed with each of the 4 bacteriophages and, after sequencing of the viral genome and bioinformatics analysis, it could be seen that all 4 were in fact the same bacteriophage. This is very interesting, as bacteriophages usually have a very limited host range, most of them are strain-specific. In this case, it is the bacteriophage of SEQ ID NO: 1, called Phi-NF1, with a very broad host spectrum, capable of infecting different genera such as *Nitrosospira* and *Nitrosomonas.*

### Infectivity in other species

After demonstrating the infective capacity of these bacteriophages, observing under the microscope that they were identical in morphology and demonstrating by sequencing that it is a single bacteriophage with a broad host spectrum for ammonium-oxidising bacteria, the possibility of infecting new nitrifying species with this Phi-NF1 phage was raised.

For this purpose, other ammonium-oxidising species such as *Nitrosomonas nitrosa* (DSM 28438) and *Nitrosomonas communis* (DSM 28336) were tested by infecting them with the phage under study and monitoring the evolution of ammonium in the culture medium. This evolution of ammonium in the culture medium is another way of seeing whether or not the virus is infecting the nitrifying bacteria being tested, which is why this test is also carried out with the species tested previously (*Nitrosomonas europaea* and *Nitrosospira multiformis*) by means of the evolution of pH.

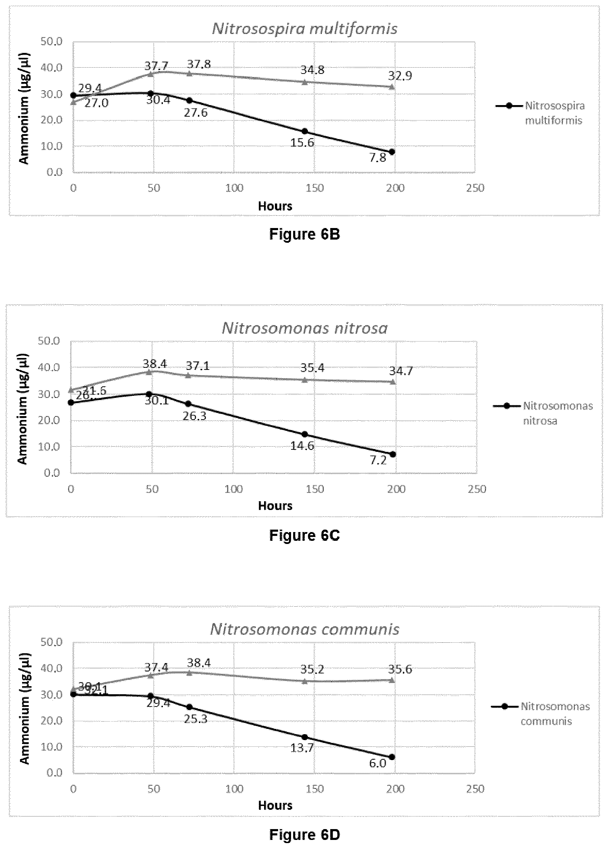

As can be seen in figures 6A, 6B, 6C and 6D, the application of a dose of 10⁷ units of Phi-NF1/ml completely eliminates ammonium consumption from the medium. This is because it eliminates each of the species tested in the different experiments.

### Infective dose of Phi-NF1 bacteriophage in vitro

Since AOB bacteria do not multiply as easily as other micro-organisms and also present great difficulty to grow in solid medium, an experiment similar to the technique known as MPN ("most probable number") was carried out to calculate the infective potential of the phage *in vitro,* starting from a concentrate with 10⁸ Phi-NF1 units/ml (approximate count carried out by transmission electron microscopy). This concentrate resulted from concentrating 10 times the phage suspension present in a culture of *Nitrosomonas europaea* previously infected by Phi-NF1 phage. The assay was performed in sterile tubes containing 20 ml of *Nitrosomonas europaea* exponential growth phase culture at pH 8.

As a control, a tube was used where the bacteria continued to multiply freely (N.e.). Phi-NF1 phage, a direct sample and decimal dilutions of the phage were added to the remaining tubes, so that the phage is successively diluted 10-fold in each tube, from a direct sample with 2 ml of 10⁸ phage in 20 ml of culture (the final concentration of phage in the tube is 10⁷ Phi-NF1 units/ml), up to dilution -8, where there should be no bacteriophage at all.

Tubes where there is no infection should have a pH very similar to the pH of the control tube (around pH 6), while tubes where viral infection has occurred would have a pH very close to 8 (between 7.5 - 8.2). The results of this test are shown in table 3 below:

**Table 3: Nitrosomonas europaea in exponential growth phase (20 ml)**

| N.s. + 2 ml Phi-NF1 | | | | | | | | | |
|---|---|---|---|---|---|---|---|---|---|
| PFU/ml | 10⁷ | 10⁶ | 10⁵ | 10⁴ | 10³ | 10² | 10 | 1 | 0 |
| Infection | Yes | Yes | Yes | Yes | Yes | Yes | Yes | No | No |

The result of this phage infectivity test shows that Phi-NF1 is able to infect *N. europaea* culture at concentrations as low as 10 Phi-NF1 units/ml.

Tubes where there is no infection will have a pH very similar to the pH of the control tube (around pH 6), while tubes where viral infection has occurred will have a pH very close to 8 (between 7.5 - 8.2).

Therefore, Phi-NF1 not only has a broad host spectrum for ammonium-oxidising nitrifying bacteria, but also has a significant infective potential, being able to infect *N*. *europaea* in vitro at a viral concentration of 10 Phi-NF1 units/ml. (This test was repeated twice with each of the nitrifying bacteria mentioned above, giving identical results).

### In vivo assay of Phage Phi-NF1 activity as a biological nitrification inhibitor

### Soil testing

To test the effect of the phage Phi-NF1 as an ecological inhibitor of nitrification, a first experiment (in triplicate) is carried out in 2 sterile containers (A and B) with 100 g of soil (in which there is a significant nitrifying activity) in each one.

Fifteen mg of ammonium sulphate (150 mg/kg) is added to both containers. In addition, 6 ml of a phage suspension of Phi-NF1 (60 ml/kg) is added in vessel 'B'.

After treatment of both soils with ammonium sulphate and soil B with the bacteriophage suspension, the concentration of NH₄⁺ and NO₃- is measured in triplicate for each point for 29 days (each point is the average of the three data). Soil A will serve as a control for the nitrifying activity of the soil.

### Experimental conditions:

- 25°C
- One sterile container with 100 g of soil for control "A" and one for test "B".
- Soil treatment (A and B) with 15 mg ammonium sulphate per 100 g soil (150 mg/kg).
- Container "B" is further treated with 6 ml of Phi-NF1 phage suspension (60 ml/kg). The phage suspension has a titre of 10⁷ PFU/ml.
- The soil samples to be analysed are collected at a depth of between 1 and 5 cm.

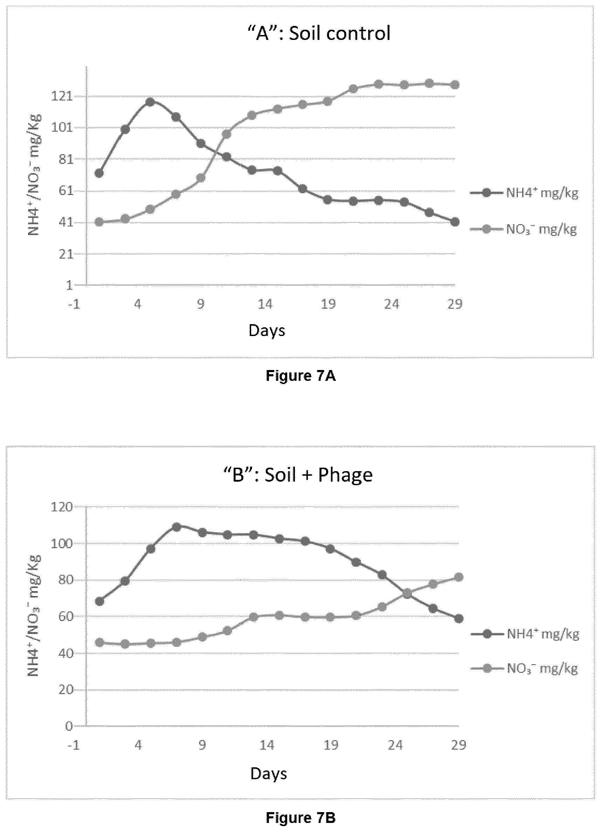

### The results are shown in figure 7.

As can be seen from the graphs in figure 7, the bacteriophage Phi-NF1 is effective as an ecological inhibitor of nitrification, in particular of ammonium-oxidising bacteria.

In the control sample "A", it can be observed how, initially, the treatment with ammonium sulphate triggers the concentration of NH₄⁺ up to 117.33 mg/Kg on day 5; however, from this moment on, the concentration of NH₄⁺ starts to decrease sharply, while the concentration of NO₃⁻ increases. This is due to the action of ammonium-oxidising and nitrite-oxidising bacteria and archaea. From day 11 onwards, the concentration of nitrate already exceeds that of ammonium (96.8 mg/kg ammonium versus 82.37 mg/kg nitrate). From this point until the end of the experiment (day 29), the ammonium concentration continues to decrease, while the nitrate curve behaves in the complete opposite way, increasing almost until the end of the experiment.

On the other hand, in sample "B" of soil + bacteriophage Phi-NF1, both ammonium and nitrate concentration behave differently from the control. At the beginning, the treatment with ammonium sulphate causes an increase in the ammonium curve similar to the control, however, the nitrate concentration remains constant during the first days. When the ammonium level reaches the highest on day 7 (108.80 mg/kg), it starts to decrease, but very slowly, without the abrupt ammonium decrease that can be seen in the control. Likewise, nitrate levels increase very slowly. This behaviour is due to the action of the bacteriophages Phi-NF1, capable of lysing and destroying an important part of the bacterial population capable of oxidising ammonium into nitrite. Up to day 25 of the experiment, nitrate levels do not exceed ammonium levels, with a difference of 14 days compared to the control test.

It seems clear that the bacteriophage Phi-NF1 acts efficiently as an ecological inhibitor of nitrification, indirectly facilitating, by lysis of an important part of the ammonium-oxidising bacteria, a greater presence of ammonium and for more days.

## Claims

1. An isolated bacteriophage selected from the group consisting of the bacteriophage of SEQ ID NO: 1 or a variant thereof, the variant retaining the phenotypic characteristics of the bacteriophage from which it is derived.

2. The bacteriophage according to claim 1, wherein the variant has at least 70% sequence identity to SEQ ID NO: 1.

3. The bacteriophage according to claim 1 or 2, **characterised in that** it inhibits the proliferation and/or growth of ammonia-oxidising bacteria (AOB) selected from *Nitrosomonas sp, Nitrosomonas aestuarii, Nitrosomonas communis, Nitrosomonas europaea, Nitrosomonas eutropha, Nitrosomonas halophila, Nitrosomonas marina, Nitrosomonas nitrosa, Nitrosomonas oligotropha, Nitrosomonas ureae, Nitrosospira sp, Nitrosospira multiformis, Nitrosospira tenuis.*

4. A use of a bacteriophage according to any one of the preceding claims as a biological nitrification inhibitor.

5. The use of a bacteriophage according to any of the preceding claims by fertigation at a dose of between 10⁸ and 10¹³ Phi-NF1 units/litre of irrigation water.

6. The use of a bacteriophage according to any one of claims 1 to 4 in a liquid or solid nitrogen fertiliser formulation at a dose between 10¹¹ and 10¹⁶ Phi-NF1 units/kg nitrogen.
